Europäisches Patentamt

European Patent Office

Office européen des brevets

(11) Publication number: **0 458 979 A1**

# EUROPEAN PATENT APPLICATION
## published in accordance with Art. 158(3) EPC

(21) Application number: 91900332.7

(22) Date of filing: **12.12.90**

(86) International application number:
PCT/JP90/01623

(87) International publication number:
WO 91/09110 (27.06.91 91/14)

(51) Int. Cl.5: **C12C 11/04**, C12G 1/02,
C12G 3/02, C12P 7/06,
C12M 1/00

(30) Priority: 15.12.89 JP 325726/89

(43) Date of publication of application:
04.12.91 Bulletin 91/49

(84) Designated Contracting States:
DE FR GB IT

(71) Applicant: KIRIN BEER KABUSHIKI KAISHA
26-1, Jingumae 6-chome
Shibuya-ku Tokyo 150(JP)

(72) Inventor: SUZUKI, Akira Kirin Beer Kabushiki
Kaisha
26-1, Jingumae 6-chome Shibuya-ku
Tokyo 150(JP)
Inventor: SUEKI, Masaaki Kirin Beer Kabushiki
Kaisha
26-1, Jingumae 6-chome Shibuya-ku
Tokyo 150(JP)
Inventor: ARIKAWA, Keiichi Kirin Beer
Kabushiki Kaisha
26-1, Jingumae 6-chome Shibuya-ku
Tokyo 150(JP)
Inventor: KOBAYASHI, Noritaka Kirin Beer
Kabushiki Kaisha
26-1, Jingumae 6-chome Shibuya-ku
Tokyo 150(JP)
Inventor: EHARA, Takashi
Kirin Beer Kabushiki Kaisha 26-1, Jingumae
6-chome
Shibuya-ku Tokyo 150(JP)

(74) Representative: Riebling, Peter, Dr.-Ing.,
Patentanwalt
Rennerle 10, Postfach 31 60
W-8990 Lindau/B.(DE)

(54) CONTINUOUS FERMENTATION PROCESS AND REACTOR THEREFOR.

(57) A continuous fermentation process which comprises supplying additional starting material stepwise into an upstream zone of a starting material solution, which flows from an inlet (3) of starting material toward an outlet (4) of product in a reaction tank (1) packed with a carrier (2) containing a microorganism immobilized thereon, in such a manner that the substrate concentration in the tank (1) is kept on a high level suitable for effecting microbial reaction so that the starting material component will have completed its reaction near the outlet (4) of the tank (1) and a desired alcohol concentration is reached. A reactor for continuous fermentation which is equipped with at least one inlet (5,6) for supplying additional starting material into an upstream zone of a starting material solution, which flows from an inlet (3) of starting material toward an outlet (4) of product in a reaction tank (1) packed with a carrier (2) containing a microorganism immobilized thereon, and which has, in the tank (1), a reaction promoting zone (7) for keeping the substrate concentration on a high level suitable for effecting microbial reaction and a reaction completing zone (8) for completing the reaction of remaining starting material so that a desired alcohol concentration is reached near the outlet of the tank.

FIG.1

## TECHNICAL FIELD

The present invention relates primarily to a method for the continuous fermentation method for the production of a high alcohol concentration, and a reactor used with this method.

## BACKGROUND ART

One conventionally known method used instead of a method where there is the supply of fixed quantities (the batch method), for the continuous and high-performance production of alcoholic beverages by brewing, is a continuous production method that produces alcohols such as ethanol by producing a microorganism reaction while continuously supplying ingredients, immobilizing the yeast to an immobilized carrier comprising alginic acid and ceramic, and the like, and using a microorganism reactor (a bioreactor) that produces alcohols while passing the ingredients through this immobilized carrier.

When the production of high-concentration alcohols (10 wt/wt% or more) is performed using such a bioreactor, it is necessary to feed the ingredients for the high alcohol concentration. When this is performed for an assumed case of producing a product that has a 15% alcohol content, it is necessary to feed ingredients that have a substrate content of approximately 30%.

However, since ingredients that have a high alcohol concentration have a high osmotic pressure, there is much damage to the yeast and the bioreaction stops, or if it does not, then the speed of reaction becomes extremely slow.

Because of this, in the production of alcohols by the continuous operation of a bioreactor, the alcohols concentration is held at a maximum of 10% and so currently, bioreactors are not used for the continuous production of products that have a high alcohol content (of 10% or more).

In the light of this, the present invention has as an object the provision of a method for continuous fermentation for the continuous production of an alcohol solution having a high concentration, and a reactor for use with this method.

## DISCLOSURE OF INVENTION

The present invention relates to a continuous fermentation method characterized in supplying to the upstream portion of a base liquid that is flowing from the ingredient inlet in the direction of the product outlet of a reactor chamber that is filled with the immobilized carrier of microorganisms, additional ingredients in stages so that the substrate concentration inside a reactor chamber is maintained within a range of a high substrate concentration suitable for the bioreaction of the substrate, so that the reaction of the ingredient components is completed in the vicinity of the product outlet of the reactor chamber and so that a predetermined concentration of alcohols is attained.

In addition, the present invention also relates to a continuous fermentation reactor that is provided with at least one additional ingredient inlet for the supply to the upstream portion of a base liquid that is flowing from the ingredient inlet in the direction of the product outlet of a reactor chamber that is filled with the immobilized carrier of microorganisms, a reaction promotion region to maintain the substrate concentration at a high substrate concentration suitable for bioreaction, and a reaction completion region provided so that the remaining ingredient components attain a predetermined alcohol concentration in the vicinity of the product outlet of the reactor chamber.

With the present invention, the supply in stages of the additional ingredients to the upstream side in the vicinity of the ingredient inlet of the reactor chamber returns the substrate concentration in the reaction promotion region for reacting with the microorganisms that have been immobilized in the reactor, to a predetermined ingredient concentration, while in the later reaction completion region, the concentration of formed alcohols drops gradually between until a predetermined value is attained, and is consumed. In this manner, additional ingredients are continuously supplied while ingredients are being supplied from the ingredient inlet of the reactor, thus making it possible to continuously obtain highly concentrated alcohols from the product outlet.

## BRIEF DESCRIPTION OF DRAWINGS

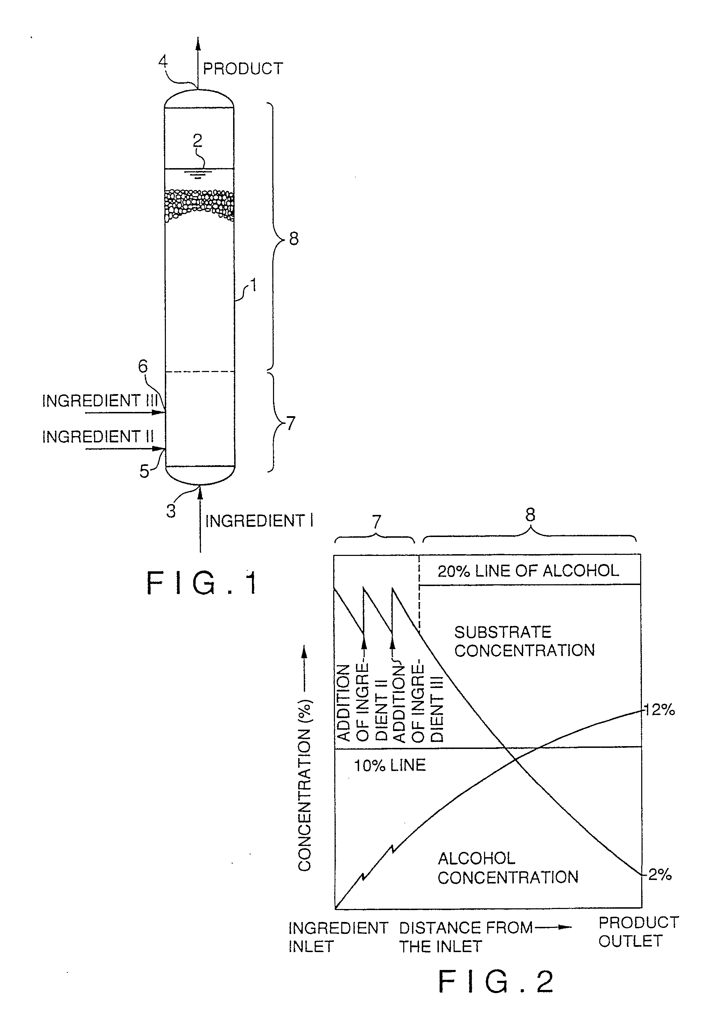

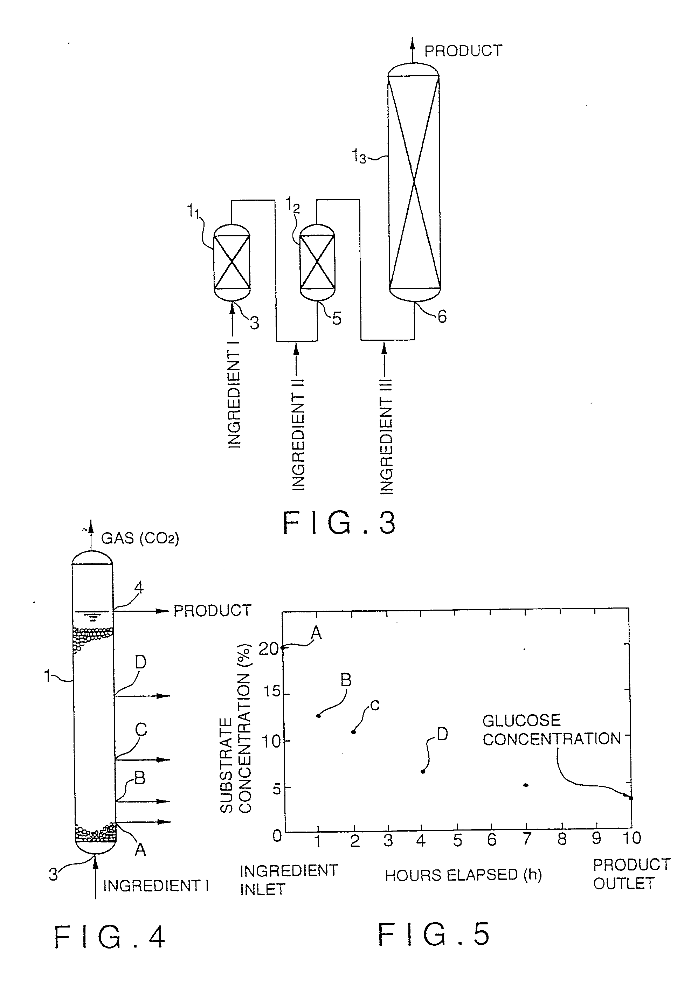

FIG. 1 is a view describing the principle of the present invention; FIG. 2 is a graph showing the relationship between the ingredient substrate concentration and the alcohol concentration; FIG. 3 is a view showing a specific configuration of the present invention; FIG. 4 is a view showing the position for checking the concentration of the ingredient substrate; FIG. 5 is a graph showing the checked substrate concentra-

3

tion; FIG. 6 is a view showing another example of a specific concentration of the present invention; FIG. 7 is a view showing yet another example of a specific concentration of the present invention; and FIG. 8 is a graph showing the change with time of the alcohol concentration in this embodiment.

BEST MODE FOR CARRYING OUT THE INVENTION

The following is a description of the continuous fermentation method and the reactor of the present invention, with reference to the appended drawings.

FIG. 1 is a view describing the principle of the present invention and FIG. 2 is a graph showing the relationship between the ingredient substrate concentration and the alcohol concentration.

Internal to the reactor 1 is a microorganism immobilized carrier 2 (such as Hypermics® of Kirin Breweries) comprising ceramic or foamed glass particles, and to a lower portion and an upper portion of the reactor 1 are provided product outlets 4.

The additional ingredient inlets 5, 6 in the upstream region in the vicinity of the ingredient inlet 3 of the reactor 1 are provided with stages along the direction of flow of the base liquid. The positions for the provision of these additional ingredient inlets 5, 6 is such that the additional ingredients are supplied in stages from the ingredient inlet 3 in volumetric ratio such as 1/10 to 3/10 on the downflow side. The reactor 1 has a reaction promotion region 7 that maintains the substrate concentration of the ingredients within the high substrate concentration range that is suitable for bioreaction, and a reaction completion region 8 that causes the remaining ingredient components to react and attain a predetermined alcohol concentration in the vicinity of the product outlet 4 of the reactor 1.

Here, the reactor 1 has a substrate concentration (of approximately 15 to 20%) for which the reaction proceeds quickly, and the ingredients II, III generally have a higher substrate concentration (30 to 50%) than that of the ingredients I.

FIG. 2 is a graph showing the relationship between the ingredient substrate concentration and the alcohol concentration. In FIG. 2, the ingredients I having a concentration of approximately 20 wt% are supplied at 10 lit./hr and the ingredients II and III having concentrations of 50 wt%, are supplied at 1.5 lit./hr from the ingredient inlet 3 of the reactor 1 by the additional ingredient inlets 5, 6 that are at positions separated by 1/10 along the length of the reactor 1.

By this, the additional ingredients II that have a high concentration are supplied from the ingredient inlet 5 in the vicinity of the ingredient inlet 3 for which the reaction speed is faster, and the concentration of the substrate inside the reactor 1 and that has dropped because of the reaction returns to the 20% line. Furthermore, the supply from the ingredient inlet 6 of additional ingredients III having a high concentration returns the substrate concentration to the 20% line, and the high substrate concentration is held at the reaction promotion region 7 and after this, the reaction is completed in the reaction completion region 8 for until the substrate concentration drops to about 2%, and there is alcohol having a concentration of 12%.

[Embodiment 1]

FIG. 3 is a view showing a specific configuration of the present invention, and the reactor is configured from first and second reactors $1_1$ and $1_2$ and from a final reactor $1_3$. Then, these are connected in a line and the ingredients I are supplied from the ingredient inlet 3 of the first reactor $1_1$, additional ingredients II are supplied from the additional ingredient inlet 5 of the second reactor $1_2$ and additional ingredients III are supplied from the ingredient inlet 6 of the final reactor $1_3$. Here, the volumes of the first and second reactors $1_1$ and $1_2$ is made within the range of 1/10 to 3/10 of the total volume.

According to this embodiment, the feed for the first stage can continuously produce highly concentrated ethanol (approx. 12 to 15%) that was previously not possible. It is necessary to have a substrate concentration of approximately 30% in order to obtain ethanol with such a high, concentration but at this concentration, there is no activation of regular yeast. Because of this, holding the substrate concentration of the ingredients within the range (approx. 13 to 20% or more desirably 13 to 18%) for which the bioreaction proceeds quickly enables the reaction ratio to be raised within an extremely short time.

[Embodiment 2]

As shown in FIG. 4 a prior test facility has an upright reactor 1 having a length of 500 mm and a diameter of 50 mm. Tests were performed with glucose liquid with a glucose concentration of 20% as the ingredient I, supply being performed from the ingredient inlet 3 at the lower portion at a ingredient speed of 0.1 lit./hr, with the average retention time being 10 hours and with Saccharaomyces cerevisiae as the microorganism used. In the first embodiment, the reactor 1 is provided with four inlets at the sampling positions A, B, C, D between the ingredient inlet 3 and the product outlet 4, and the base liquid was extracted from these and the glucose concentrations measured for each of the positions A, B, C, D.

As the result of the test described above, the glucose concentration was 20% at the sampling position A in the vicinity of the ingredient inlet 3 shown in FIG. 5, and had reduced to approximately 13% after one hour had elapsed, to approximately 11% after 2 hours had elapsed, to approximately 7% after 4 hours had elapsed, to approximately 5% after 7 hours had elapsed, and to approximately 6% at the product outlet 4.

From these results, it is clear that after the passage of one hour, the substrate (glucose) is quickly consumed at the approximately 1/10 position of the reactor 1, when converted into volumes, and has been changed into ethanol.

[Embodiment 3]

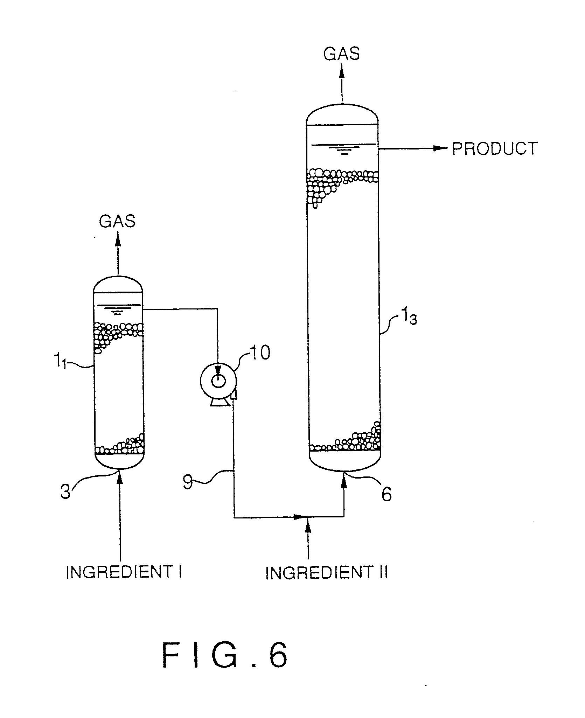

On the basis of the tests described above, the test apparatus shown in FIG. 6 was used in a status suited to production. More specifically, the first reactor $1_1$ and the final reactor $1_3$ were used, with the first reactor $1_1$ being made of glass and having a length of 200 mm, a diameter of 40 mm, and a volume of 0.25 lit, with the outlet of the first reactor $1_1$ and the final reactor $1_3$ also being made of glass and having a length of 570 mm, a diameter of 50 mm, and a volume of 1.1 lit., with a pump 10 being provided along the pipe 9 that connects the outlet of the first reactor $1_1$ and the inlet of the final reactor $1_3$. In addition, the ingredients I having a glucose concentration of 20% are supplied at a flow rate of 55 cc/h from the ingredient inlet 3 of the first reactor $1_1$, and additional ingredients II having a glucose concentration of 45% are supplied at a flow rate of 35 cc/h from the ingredients inlet 6 along the pipe downstream from the pump 10. The microorganism used was Saccharaomyces cerevisiae.

The total retention time according to the test described above was as follows.

$$T = \frac{1.1 + 0.25}{(0.055 + 0.035)} = 15 \ (h)$$

The total amount of added substrate was as follows.

$$C = \frac{(55 \times 0.2) + (35 \times 0.45)}{(55 + 35)} = 29.7 \ (\%)$$

From tests using the apparatus described above, it was possible to continuously produce a product having an ethanol content of 14.1% for 76 days despite the retention time being less than 15 hours.

With the test apparatus described above, feed was performed for two times but the results described above could be improved if there are three or more stages of feed.

[Embodiment 4]

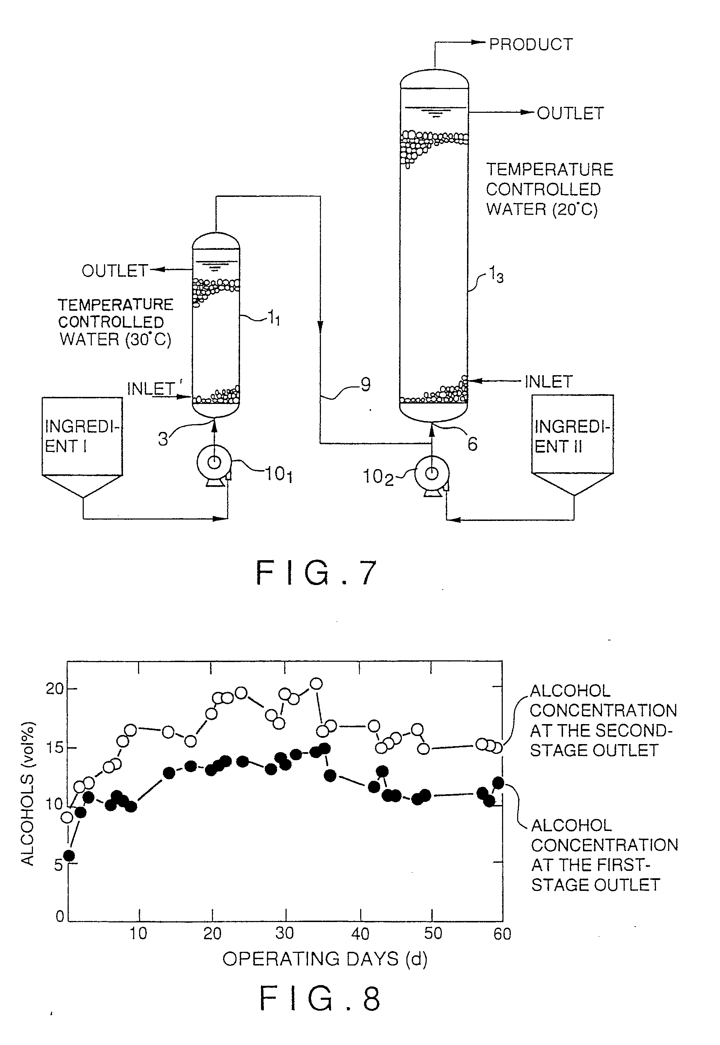

The tests for the second embodiment were used as the basis for the implementation of a production status using the test apparatus shown in FIG. 7. More specifically, the first reactor $1_1$ and the final reactor $1_3$ were used, with the first reactor $1_1$ being made of glass and having a length of 240 mm, a diameter of 40 mm, and a volume of 0.304 lit, the outlet of the first reactor $1_1$ and the final reactor $1_3$ being made of glass and having a length of 570 mm, a diameter of 50 mm and a volume of 1.04 lit., and a pump 10 being provided along a pipe 9 connecting the outlet of the first reactor $1_1$ and the final reactor $1_3$, and with hot water being used to adjust the temperatures of the first reactor $1_1$ and the final reactor $1_3$ to $30°C$ and $20°C$ respectively.

In addition the ingredients I having a glucose concentration of 20% were supplied from the ingredients inlet 3 of the first reactor $1_1$ at a rate of 6 cc/h by the flow pump $10_1$, and additional ingredients II having a concentration of 30 to 35% were supplied at a flow rate of 10 cc/h to the pip 9 downstream from a pump $10_2$. The microorganism used was Saccharaomyces cerevisiae.

The total retention time according to the test described above was as follows.

$$T = \frac{1.04 + 0.304}{(0.006 + 0.01)} = 84 \ (h)$$

The total amount of added substrate was as follows.

$$C = 100 \times \frac{(6 \times 0.2) + (10 \times 0.325)}{(6 + 10)} = 27.8 \ (\%)$$

From the tests using the test apparatus described above, it was possible to continuously produce a product having an ethanol concentration of more than 15.1%, for a period of two months.

More specifically, as the result of the tests described above, as shown in FIG. 8 for the change with time of the ethanol concentration in this embodiment, the test apparatus was operated for approximately two months, and ethanol at a concentration of 10 to 13% volume was continuously produced at the first-stage outlet of the reactor 1 and at the second-stage outlet of the final reactor $1_3$ that received this, ethanol at a concentration of 15 to 20% volume was continuously obtained.

## INDUSTRIAL APPLICABILITY

As has been described above, the present invention supplies additional ingredients in stages in the upstream flow region of base liquid that flows from the ingredient inlet of the reactor tank filed with a microorganism immobilized carrier and towards the product outlet, so that the substrate concentration inside the reactor is held at a level suitable for bioreaction, and the reaction of the ingredient components is completed in the vicinity of the product outlet of the reactor to attain a predetermined alcohol concentration. As a result, it is possible to have the continuous production of uniform and highly concentrated alcohol in a short time and using a small-scale facility, so that the alcohol production ratio is increased remarkably to make this continuous fermentation method and the reactor for it extremely effective.

## Claims

1. A continuous fermentation method comprising the steps of:
   supplying to the upstream portion of a base liquid that is flowing from an ingredient inlet in the direction of a product outlet of a reactor chamber that is filled with a microorganism immobilized carrier, additional ingredients in stages so that the substrate concentration inside a reactor chamber is maintained within a range of a high substrate concentration suitable for the bioreaction of the substrate,
   completing the reaction of the ingredient components in the vicinity of the product outlet of the reactor chamber, and
   attaining a predetermined concentration of alcohol.

2. The continuous fermentation method of claim 1, wherein:
   a substrate concentration range suitable for said bioreaction is 13 to 20 wt%.

3. A continuous fermentation reactor, comprising: at least one additional ingredient inlet for the supply to the upstream portion of a base liquid that is flowing from a ingredient inlet in the direction of a product outlet of a reactor chamber that is filled with a microorganism immobilized carrier,
   a reaction promotion region internal to the reactor to maintain the substrate concentration at a high substrate concentration suitable for bioreaction, and
   a reaction completion region provided so that the remaining ingredient components attain a predetermined alcohol concentration in the vicinity of the product outlet of the reactor chamber.

4. The continuous fermentation reactor of claim 4, wherein:
   said reactor comprises a plural number of reactors that are connected so as to be freely feed, with ingredients being supplied to the reactor that is furthest upstream, and additional ingredients being supplied to the reactors downstream.

FIG.1

FIG.2

PRODUCT

FIG.3

GAS (CO₂)

PRODUCT

INGREDIENT I

FIG.4

SUBSTRATE CONCENTRATION (%)

GLUCOSE CONCENTRATION

INGREDIENT INLET

HOURS ELAPSED (h)

PRODUCT OUTLET

FIG.5

GAS

PRODUCT

GAS

$1_3$

$1_1$

10

9

3

6

INGREDIENT I

INGREDIENT II

# FIG.6

FIG.7

FIG.8

# INTERNATIONAL SEARCH REPORT

International Application No PCT/JP90/01623

## I. CLASSIFICATION OF SUBJECT MATTER (if several classification symbols apply, indicate all) 6

According to International Patent Classification (IPC) or to both National Classification and IPC

Int. Cl$^5$ C12C11/04, C12G1/02, C12G3/02, C12P7/06, C12M1/00

## II. FIELDS SEARCHED

| Minimum Documentation Searched 7 | |
|---|---|
| Classification System | Classification Symbols |
| IPC | C12C11/00-13/06, C12G1/00-3/02, C12P7/06, C12M1/00 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| Y | JP, A, 59-154981 (USA), September 4, 1984 (04. 09. 84) | 1-4 |
| Y | JP, A, 55-26857 (Takinosuke Furuichi), February 26, 1980 (26. 02. 80), (Family: none) | 1-4 |
| A | JP, A, 64-43184 (Ozeki Kagaku Kogyo K.K.), February 15, 1989 (15. 02. 89), (Family: none) | 1-4 |

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| March 6, 1991 (06. 03. 91) | March 25, 1991 (25. 03. 91) |
| International Searching Authority | Signature of Authorized Officer |
| Japanese Patent Office | |

Form PCT/ISA/210 (second sheet) (January 1985)